(19) Europäisches Patentamt / European Patent Office / Office européen des brevets

(11) **EP 3 338 632 B1**

(12) **FASCICULE DE BREVET EUROPEEN**

(45) Date de publication et mention
de la délivrance du brevet:
**28.08.2019 Bulletin 2019/35**

(51) Int Cl.:
***A61B 5/1455*** (2006.01)

(21) Numéro de dépôt: **17210569.4**

(22) Date de dépôt: **26.12.2017**

(54) **DÉTERMINATION D'UNE TENEUR EN OXYGÈNE D'UN TISSU CORPOREL VIVANT**

BESTIMMUNG DES SAUERSTOFFGEHALTS IN EINEM LEBENDEN KÖRPERGEWEBE

DETERMINATION OF AN OXYGEN CONTENT OF A LIVING BODY TISSUE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **26.12.2016 FR 1663395**

(43) Date de publication de la demande:
**27.06.2018 Bulletin 2018/26**

(73) Titulaire: **COMMISSARIAT À L'ÉNERGIE
ATOMIQUE ET AUX
ÉNERGIES ALTERNATIVES
75015 Paris (FR)**

(72) Inventeurs:
• **GERBELOT BARILLON, Rémi
38450 VIF (FR)**
• **KOENIG, Anne
38410 SAINT MARTIN D'URIAGE (FR)**

(74) Mandataire: **GIE Innovation Competence Group
310, avenue Berthelot
69372 Lyon Cedex 08 (FR)**

(56) Documents cités:
WO-A1-2013/166461     US-B1- 7 884 933
US-B1- 9 498 134

**Description**

## DOMAINE TECHNIQUE

Le domaine technique de l'invention est la détermination d'une teneur en oxygène du sang circulant dans un tissu corporel vivant, humain ou animal, par un procédé optique.

## ART ANTERIEUR

La détermination de la saturation d'oxygène à l'aide de moyens optiques est une technique mature, et déclinée dans des dispositifs, dits d'oxymétrie, couramment utilisés en milieu hospitalier, dans des cabinets médicaux ou à domicile. Les principes mis en oeuvre sont bien connus. Il s'agit d'illuminer un tissu vascularisé, en particulier l'extrémité d'un doigt, successivement à l'aide de deux longueurs d'onde, dans le rouge (typiquement 660 nm) et dans l'infrarouge (typiquement 940 nm). On mesure un rayonnement optique transmis par le tissu à chaque longueur d'onde, ce qui permet de mesurer une absorption du tissu à chaque longueur d'onde. Du fait de la pulsation cardiaque, l'absorption à chaque longueur d'onde fluctue entre une valeur basse, dite valeur de base, et une valeur haute, dite valeur pulsatile. La pression partielle d'oxygène est alors établie à partir de ratios de l'absorption pulsatile et l'absorption de base respectivement calculés dans le rouge et dans l'infrarouge. Les applications sont courantes dans des configurations dites en transmission, dans lesquelles le tissu analysé est disposé entre des sources lumineuses et un photodétecteur.

L'émergence de dispositifs nomades, portés par la personne, a entraîné le développement de dispositifs fonctionnant selon une configuration en rétrodiffusion, les sources lumineuses et le photodétecteur étant adjacents, et faisant face au tissu à caractériser. Le photodétecteur mesure alors un rayonnement optique rétrodiffusé par le tissu. Citons par exemple la publication Haahr R.,"An Electronic Patch for Wearable Health Monitoring by Reflectance Pulse Oxymetry", IEEE Transactions on biomedical circuits and systems, 2011. Cette publication décrit un dispositif comportant deux sources lumineuses centrées par rapport à une photodiode annulaire. Le dispositif est intégré dans un patch, de façon à pouvoir être porté par une personne, pour effectuer une surveillance en continu et de longue durée. Le brevet US8055321 décrit également un dispositif d'oxymétrie selon une configuration de rétrodiffusion.

Le document US9498134 décrit un dispositif optique de détermination de propriétés optiques d'un tissu corporel, mettant en oeuvre trois distances de rétrodiffusion différentes. Mais le dispositif nécessite l'utilisation de trois longueurs d'ondes différentes.

Un inconvénient des dispositifs d'oxymétrie tels que décrits dans les documents précédemment cités est la nécessité d'un contact permanent et stable entre les moyens optiques, c'est-à-dire les sources lumineuses et le photodétecteur, et le tissu. Des difficultés peuvent notamment survenir lorsque le porteur du dispositif est en mouvement. La position des moyens optiques vis-à-vis de la surface de la peau du porteur peut alors varier, ce qui cause des erreurs de mesure. Par ailleurs, certaines caractéristiques des dispositifs actuels, en particulier la longueur d'onde des sources lumineuses, peuvent être optimisées. L'invention décrite ci-après répond à ces problèmes.

## EXPOSE DE L'INVENTION

Un premier objet de l'invention est un procédé d'estimation d'une teneur en oxygène dans un tissu corporel vivant, comportant les étapes suivantes:

a) illumination d'une surface du tissu par un faisceau lumineux incident, selon une longueur d'onde d'intérêt, de façon à former une zone élémentaire d'illumination, correspondant à la partie de la surface éclairée par le faisceau lumineux incident;
b) au cours de l'illumination, détection simultanée d'un rayonnement rétrodiffusé émanant de la surface de l'échantillon au niveau d'au moins trois zones élémentaires de détection, chaque zone élémentaire de détection respectivement s'étendant à une distance, dite distance de rétrodiffusion, de la zone élémentaire d'illumination, chaque distance de rétrodiffusion étant différente l'une de l'autre et inférieure à 15 mm;
c) comparaisons, deux à deux, des intensités des rayonnements rétrodiffusés détectés, ainsi que des distances de rétrodiffusion ;
d) prise en compte de termes de calibration déterminés au cours d'une phase de calibration ;
e) estimation d'une teneur en oxygène du tissu, à partir des comparaisons effectuées lors de l'étape c) et des termes de calibration pris en compte lors de l'étape d).

De préférence, lors de l'étape b), chaque distance de rétrodiffusion est supérieure à 0.3 cm. L'étape d) comporte les étapes suivantes :

di) prise en compte d'un premier terme de calibration, représentant une quantité d'hémoglobine dans le tissu observé ;
dii) prise en compte d'un deuxième terme de calibration, caractérisant une absorption du tissu par des composants du tissu autres que l'hémoglobine.

L'étape c) peut comporter les étapes suivantes :

ci) soustraction des intensités de rayonnements de rétrodiffusion détectés à deux distances de rétrodiffusion différentes;
cii) calcul d'un logarithme de dite soustraction;
ciii) soustraction de deux distances de rétrodiffusion différentes, notamment une soustraction, deux à deux, des distances de diffusion considérées.

L'étape c) comporte une détermination de deux coefficients à partir des comparaisons effectuées ; L'étape e) comporte une application d'une expression reliant la teneur en oxygène aux termes de calibration pris en compte lors de l'étape d) ainsi qu'aux coefficients obtenus lors de l'étape c), l'expression étant basée sur des valeurs connues de coefficients d'absorption de l'oxyhémoglobine et de la désoxyhémoglobine à la longueur d'onde d'intérêt.
Les termes de calibration pris en compte lors de l'étape d) sont déterminés :

soit au cours d'une phase préalable de calibration, au cours de laquelle on dispose d'une estimation de la teneur en oxygène du tissu, la phase de calibration comportant les étapes suivantes :

cal-i) mise en oeuvre des étapes a) à c), à deux longueurs d'onde différentes;
cal-ii) utilisation de l'expression appliquée lors de l'étape e) à chaque longueur d'onde, en considérant la teneur en oxygène estimée;

soit au cours d'une phase préalable de calibration, comportant les étapes suivantes :

cal-i) mise en oeuvre des étapes a) à c), à trois longueurs d'onde différentes, une desdites longueurs d'onde étant une longueur d'onde dite isobestique, à laquelle le coefficient d'absorption de l'oxyhémoglobine est considéré comme égal au coefficient d'absorption de la déoxyhémoglobine ;
cal-ii) à partir de l'expression appliquée lors de l'étape e), à chaque longueur d'onde, obtention des termes de calibration;

soit au cours d'une phase préalable de calibration, la phase de calibration comportant les étapes suivantes :

cal-i) mise en oeuvre des étapes a) à c), à trois longueurs d'onde différentes ;
cal-ii) à partir de l'expression appliquée lors de l'étape e), à chaque longueur d'onde, obtention des termes de calibration;

les étapes c) à e) étant mises en oeuvre à l'aide d'un processeur.
Un autre objet de l'invention est un procédé bimodal de détermination d'une teneur en oxygène dans un tissu corporel vivant, le procédé comportant une première détermination d'une teneur en oxygène selon une première modalité, selon les étapes suivantes

la) illumination, par exemple successive, d'une surface du tissu par un faisceau lumineux incident, selon première longueur d'onde et une deuxième longueur d'onde, de façon à former, lors de chaque illumination, une zone élémentaire d'illumination, correspondant à la partie de la surface éclairée par le faisceau lumineux incident;
1b) au cours de chaque illumination, détection d'un rayonnement rétrodiffusé émanant de la surface de l'échantillon à une même distance, dite distance de rétrodiffusion, la distance de rétrodiffusion étant inférieure à 15 mm, de façon à obtenir un rayonnement rétrodiffusé détecté respectivement à la première longueur d'onde et à la deuxième longueur d'onde;
1c) à partir de chaque rayonnement rétrodiffusé ainsi détecté, calcul d'une réflectance à la première et à la deuxième longueur d'onde ;
1d) réitération des trois étapes 1a) à 1c) de façon à obtenir une évolution temporelle de la réflectance du tissu à la première et à la deuxième longueur d'onde ;
le) détermination d'une valeur minimale périodique et d'une valeur maximale périodique de l'évolution temporelle de la réflectance à chaque longueur d'onde ;
1f) calcul d'un ratio, à chaque longueur d'onde, de la valeur maximale périodique et de la valeur minimale périodique déterminées lors de l'étape 1e) ;
1g) détermination de la teneur en oxygène du tissu à partir des ratios calculés lors de l'étape 1f) à chaque longueur d'onde ;

le procédé étant caractérisé en ce qu'il comporte une étape de validation ou d'invalidation de la teneur en oxygène déterminée lors de l'étape 1g), et en ce qu'en cas d'invalidation, la teneur en oxygène est déterminée selon une deuxième modalité, la deuxième modalité étant mise en oeuvre selon un procédé selon le premier objet de l'invention.

La première modalité est basée sur une évolution temporelle de l'intensité du rayonnement rétrodiffusé par le tissu selon deux longueurs d'onde. La deuxième modalité est basée sur une évolution spatiale du rayonnement rétrodiffusé par le tissu selon une longueur d'onde. Les inventeurs ont estimé que ces modalités peuvent être combinées et être alternativement mises en oeuvre.

Par réflectance, on entend une grandeur représentative d'une quantité de rayonnement rétrodiffusé par le tissu normalisée par une quantité de rayonnement du faisceau incident.

L'invalidation peut être effectuée lorsqu'un mouvement du dispositif est détecté, ou en cas de fluctuations jugées anormales des rayonnements rétrodiffusés détectés lors de l'étape 1b) ou en cas d'obtention, lors de l'étape 1g), d'une teneur en oxygène en dehors d'une plage d'invalidité prédéterminée.

Le procédé peut comporter l'une des caractéristiques suivantes, prises isolément ou selon les combinaisons techniquement possibles :

- La première longueur d'onde est comprise entre 730 et 750 nm, par exemple 740 nm, et dans lequel la deuxième longueur d'onde est comprise entre 860 et 880 nm, par exemple 870 nm.
- Lors de l'étape 1b), la distance de rétrodiffusion est comprise entre 6 mm et 8 mm.
- La longueur d'onde d'intérêt utilisée lors de la deuxième modalité est soit la première longueur d'onde, soit la deuxième longueur d'onde.
- L'étape 1a) est mise en oeuvre à l'aide d'une source de lumière et l'étape 1b) est mise en oeuvre à l'aide d'un photodétecteur élémentaire, la source de lumière et/ou le photodétecteur élémentaire étant solidaires d'un capteur de mouvement, de telle sorte que lorsqu'un mouvement significatif est détecté par le capteur de mouvement, la première modalité est invalidée.
- Le procédé comporte une étape de mesure d'une fréquence cardiaque relative au tissu corporel vivant, de telle sorte que lorsque la fréquence cardiaque, ou lorsqu'une variation de la fréquence cardiaque, franchit un certain seuil, la première modalité est invalidée.

Un troisième objet de l'invention est un dispositif d'oxymétrie, apte à être appliqué contre le corps d'un individu ou d'un animal, comportant :

- une première source de lumière apte à émettre une lumière dans une longueur d'onde comprise entre 600 nm et 800 nm ;
- une deuxième source de lumière apte à émettre une lumière dans une longueur d'onde comprise entre 800 nm et 1000 nm ;
- au moins trois photodétecteurs élémentaires, s'étendant à trois distances, dites distances de rétrodiffusion, d'une desdites sources de lumière, les distances de rétrodiffusion étant inférieures à 1.5 cm ;
- un processeur, apte à mettre en oeuvre les étapes c) à e) d'un procédé selon le premier objet de l'invention, à partir de rayonnements de rétrodiffusion détecté par chaque photodétecteur élémentaire durant l'activation de la première source de lumière ou de la deuxième source de lumière ;

le processeur étant également apte à mettre en oeuvre les étapes 1c) à 1g) d'un procédé selon le deuxième objet de l'invention, à partir d'un rayonnement rétrodiffusé détecté par un photodétecteur élémentaire durant l'activation de la première source de lumière et de la deuxième source de lumière.

Le dispositif peut comporter un moyen de détecter un mouvement, par exemple un capteur de mouvement, configuré pour détecter un mouvement du dispositif. Les distances de rétrodiffusion peuvent être respectivement comprises entre 3 mm et 10 mm.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

Les figures 1A, 1B et 1C représentent un exemple de dispositif selon l'invention.

La figure 2A représente les principales étapes d'un procédé de mesure d'une saturation pulsatile d'oxygène selon une première modalité. La figure 2B illustre la variation temporelle d'une réflectance à une première longueur d'onde.

La figure 3 montre l'évolution de la réflectance, une longueur d'onde de 660 nm, en fonction de la distance de rétrodiffusion.

Les figures 4A, 4B et 4C sont des résultats d'une modélisation du trajet de photons dans un tissu vascularisé soumis à une illumination par un faisceau à la longueur d'onde de 660 nm. Les figures 4D, 4E et 4F sont des résultats d'une modélisation du trajet de photons dans un tissu vascularisé soumis à une illumination par un faisceau à la longueur d'onde de 740 nm. La figure 4G représente des profils déterminés à partir des figures 4A à 4F.

La figure 5 montre les principales étapes d'un procédé de mesure d'une saturation d'oxygène selon une deuxième modalité, ainsi que les principales étapes de phases de calibration nécessaires à cette deuxième modalité.

## EXPOSE DE MODES DE REALISATION PARTICULIERS

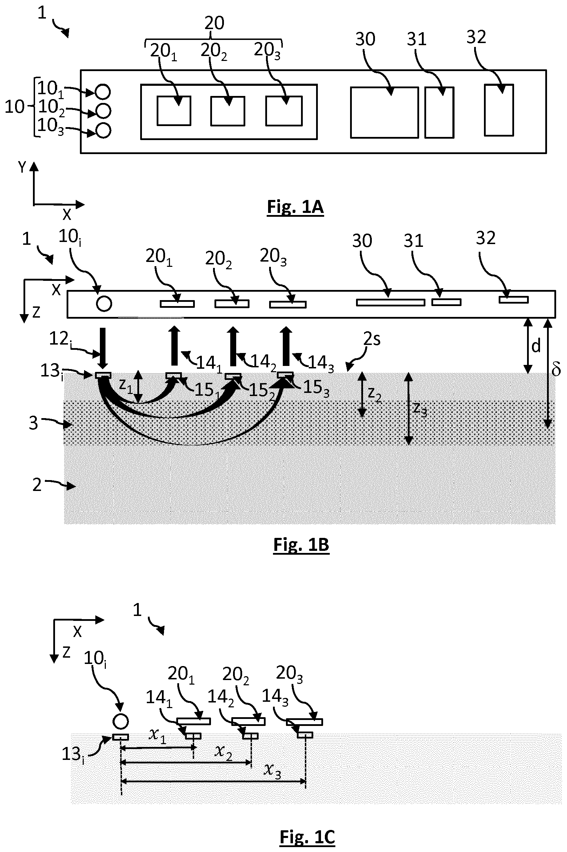

Les figures 1A à 1C représentent un exemple de réalisation d'un dispositif 1 permettant de mesurer une teneur d'oxygène dans un tissu 2 vascularisé. Le terme teneur en oxygène peut désigner une pression partielle d'oxygène dans le sang, ou une saturation d'oxygène. La saturation d'oxygène est un ratio, exprimé sous la forme d'un pourcentage, entre une concentration d'oxyhémoglobine sur la concentration d'hémoglobine totale, cette dernière étant la somme des concentrations de désoxyhémoglobine et d'oxyhémoglobine. Dans les exemples décrits ci-dessous, la teneur en oxygène correspond à la saturation d'oxygène. Le dispositif s'étend selon un plan XY, comme illustré sur la figure 1A. La figure 1B représente une coupe du dispositif et du tissu selon un plan XZ orthogonal au plan XY. La figure 1C représente une coupe du dispositif selon le plan XZ.

Le tissu 2 est un tissu corporel, contre lequel le dispositif 1 est appliqué. Il peut par exemple s'agir d'un membre supérieur, d'un torse ou d'un membre inférieur. Le dispositif 1 est appliqué contre une surface $2s$ du tissu examiné. La surface $2s$ s'étend selon le plan XY. Le tissu comporte notamment des vaisseaux ou capillaires sanguins 3 s'étendant à une certaine profondeur $\delta$ de la surface $2s$. La profondeur $\delta$ est typiquement comprise entre 100 $\mu$m et 2 ou 3 mm.

Le dispositif comporte une source de lumière 10 incluant au moins deux sources de lumière élémentaires. Dans cet exemple, on a représenté trois sources de lumière élémentaires :

- une première source de lumière élémentaire $10_1$, apte à émettre un premier faisceau lumineux $12_1$, selon une longueur d'onde rouge $\lambda_1$, dite première longueur d'onde, comprise entre 600 et 800 nm ;
- une deuxième source de lumière élémentaire $10_2$, apte à émettre un deuxième faisceau lumineux $12_2$, dans une longueur d'onde infrarouge $\lambda_2$, dite deuxième longueur d'onde, comprise entre 800 et 1000 nm ;
- une troisième source de lumière élémentaire $10_3$, apte à émettre un troisième faisceau lumineux $12_3$, dans une troisième longueur d'onde $\lambda_3$, correspondant à un point isobestique du spectre d'absorption de la désoxyhémoglobine et de l'oxyhémoglobine.

De préférence, la largeur des bandes spectrales de chaque source de lumière est inférieure à 100 nm ou à 50 nm. Par largeur de bande, on entend la largeur à mi-hauteur du pic d'émission correspondant à ladite bande spectrale.

Le dispositif comporte également un photodétecteur 20, comprenant au moins trois photodétecteurs élémentaires : un premier photodétecteur élémentaire $20_1$, un deuxième photodétecteur élémentaire $20_2$ et un troisième photodétecteur élémentaire $20_3$. Chaque photodétecteur élémentaire peut être une photodiode. Le photodétecteur 20 peut être un détecteur pixelisé, par exemple un capteur CMOS ou un capteur CCD auquel cas chaque photodétecteur élémentaire est formé par un pixel ou un groupe de pixels adjacents les uns des autres. Les pixels peuvent s'étendre selon une dimension (photodétecteur linéaire) ou deux dimensions (photodétecteur matriciel). Le dispositif comporte également un processeur 30, par exemple un microprocesseur, apte à exécuter les opérations de traitement de signal décrites ci-après. Ces opérations sont programmées dans une mémoire 31, comportant des instructions pour leur mise en oeuvre par le processeur 30.

Le dispositif peut comporter un capteur de mouvement 32, par exemple un accéléromètre ou un gyromètre pour détecter un mouvement relatif du dispositif par rapport au tissu examiné. Le capteur de mouvement peut également se baser sur des signaux fournis par les photodétecteurs élémentaires, précédemment décrits, pour détecter un mouvement. Le capteur de mouvement est notamment apte à détecter une variation de la distance d entre le dispositif 1 et le tissu examiné 2.

Chaque source de lumière élémentaire $10_i$ est apte à produire un faisceau lumineux incident $12_i$ dans une longueur d'onde $\lambda_i$ se propageant vers le tissu 2, l'indice i étant un entier désignant la longueur d'onde du faisceau incident $12_i$. L'intersection de chaque faisceau lumineux incident $12_i$ avec la surface du tissu $2s$ forme une zone élémentaire d'illumination $13_i$. Chaque zone élémentaire d'illumination $13_i$ a une forme délimitée, inscrite dans un diamètre avantageusement inférieur à 2 mm, et de préférence inférieur à 500 $\mu$m. Les sources de lumière élémentaires $10_i$ sont

impulsionnelles et peuvent être activées l'une après l'autre, selon une fréquence pouvant être égale à 100 Hz.

Sous l'effet de l'illumination impulsionnelle par chaque faisceau lumineux incident $12_i$, des photons sont injectés dans le tissu, et subissent une absorption ou une diffusion respectivement en fonction des propriétés optiques d'absorption ou de diffusion du tissu. Les termes propriétés optiques d'absorption et de diffusion désignent un ou plusieurs facteurs gouvernant respectivement l'absorption et la diffusion d'un photon dans le tissu 2 examiné. Une propriété optique d'absorption peut être un coefficient d'absorption $\mu_a$. Une propriété optique de diffusion peut être un coefficient de diffusion $\mu_s$, ou un coefficient de diffusion réduit $\mu'_s$. Dans le cas d'un tissu biologique, du fait des multiples diffusions dans le tissu 2, un rayonnement rétrodiffusé 14 se forme, émanant de l'échantillon à l'extérieur de la zone élémentaire d'illumination $13_i$. Sur la figure 1B, on a représenté des rayonnements rétrodiffusés $14_1$, $14_2$ et $14_3$ émanant du tissu à différentes distances d'une zone élémentaire d'illumination $13_i$. Les photodétecteurs élémentaires $20_1$, $20_2$ et $20_3$ sont respectivement disposés face au tissu 2, de façon à détecter un rayonnement rétrodiffusé émanant de l'échantillon au niveau d'une zone élémentaire de détection $15_1$, $15_2$ et $15_3$. Par zone élémentaire de détection $15_j$, on entend une partie de la surface 2s du tissu à travers laquelle émane un rayonnement rétrodiffusé $14_j$ détecté par un photodétecteur élémentaire $20_j$. Les photodétecteurs élémentaires sont disposés de façon à définir trois zones élémentaires de détection $15_j$ ($1 \leq j \leq 3$) distinctes l'une de l'autre, et disposées à différentes distances $x_{i,j}$, dites distances de rétrodiffusion, d'une zone élémentaire d'illumination $13_i$. Dans le dispositif représenté sur les figures 1A à 1C, du fait de l'agencement symétrique et de la proximité des sources de lumière élémentaires $11_i$, chaque distance de rétrodiffusion $x_{i,j}$ s'étendant entre une zone élémentaire d'illumination $13_i$ et une zone élémentaire de détection $15_j$ est considérée comme indépendante de la zone élémentaire d'illumination $13_i$. Autrement dit, on considère que quel que soit i, $x_{i,j} = x_j$. On a représenté, sur la figure 1C, trois distances de rétrodiffusion $x_1$, $x_2$ et $x_3$. Chaque distance de rétrodiffusion est non nulle : chaque zone élémentaire de détection est préférentiellement distante de la zone élémentaire d'illumination, chaque zone élémentaire de détection étant séparée, et non superposée, à une zone élémentaire d'illumination.

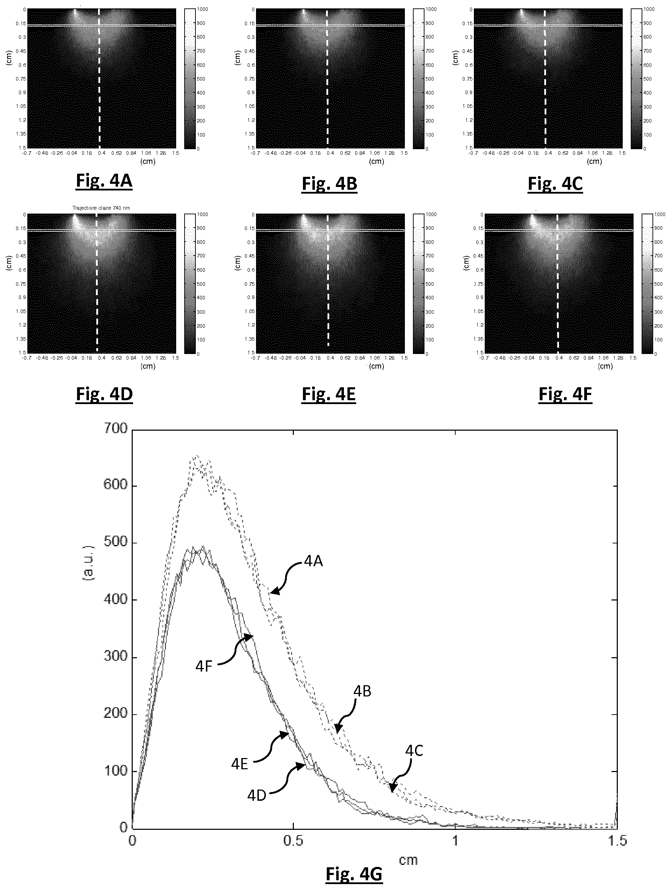

Le trajet de photons dans un tissu peut être simulé par des codes de calculs de type Monte-Carlo, comme décrit en lien avec les figures 4A à 4F. Sur la figure 1B, on a représenté, par des flèches, le trajet, dans le tissu 2, des photons contribuant aux rayonnements rétrodiffusés respectifs $14_1$, $14_2$ et $14_3$ respectivement associés aux zones élémentaires de détection $15_1$, $15_2$ et $15_3$. Plus la distance de rétrodiffusion $x_j$ augmente, moins l'intensité du rayonnement rétrodiffusé $14_j$ est élevée, mais plus la profondeur maximale $z_j$ atteinte par les photons dans le tissu 2 est importante. Une distance de rétrodiffusion $x_j$ ne doit pas être trop élevée de façon à ce que le rayonnement rétrodiffusé $14_j$ lui correspondant soit suffisamment intense pour être exploitable. C'est pourquoi il est préférable que chaque distance de rétrodiffusion soit inférieure à 1,5 cm, voire 1 cm. Une distance de rétrodiffusion $x_j$ ne doit pas être trop faible de façon à ce que le rayonnement rétrodiffusé $14_j$ lui correspondant pénètre suffisamment en profondeur dans le tissu pour être formé par des photons ayant interagi avec le sang circulant dans le tissu. Les inventeurs estiment qu'il est préférable que chaque distance de rétrodiffusion soit supérieure à 0,3 cm.

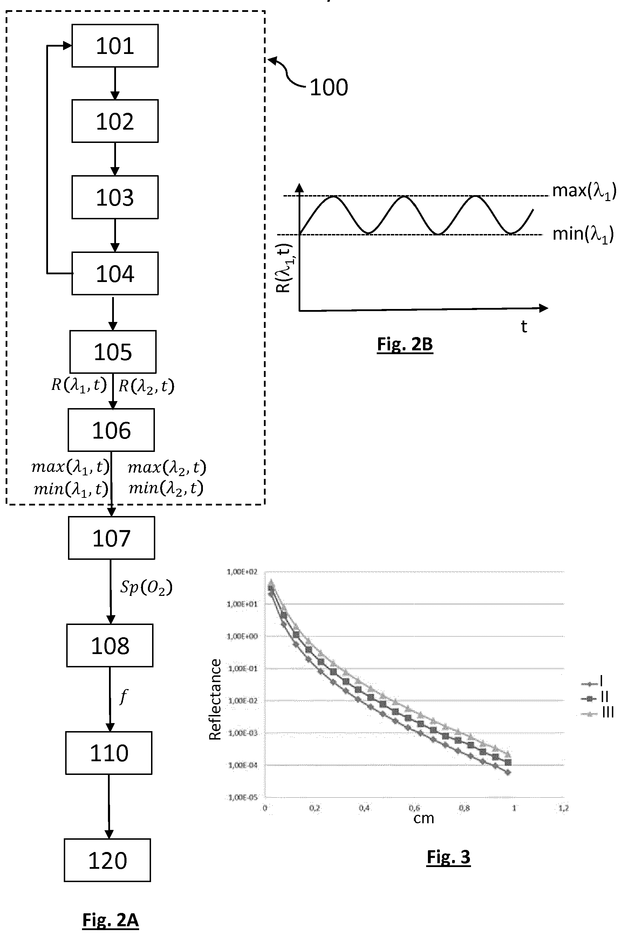

On va décrire à présent les principales étapes d'un procédé permettant d'estimer une saturation, dite pulsée, d'oxygène dans le tissu, en lien avec les figures 2A et 2B. Les principales étapes décrites en lien avec la figure 2A sont explicitées ci-dessous :

Etape 101 : première illumination : le tissu est illuminé par la première source de lumière élémentaire $10_1$ dans la première bande spectrale $\lambda_1$, les autres sources de lumière élémentaires n'étant pas activées.

Etape 102 : première détection : un rayonnement rétrodiffusé $14(\lambda_1)$ est détecté par un photodétecteur élémentaire $20_j$ dans la première bande spectrale $\lambda_1$, la détection étant synchrone de la première illumination.

Etape 103 : deuxième illumination : le tissu est illuminé par la deuxième source de lumière élémentaire $10_2$, dans la deuxième bande spectrale $\lambda_2$, les autres sources de lumière élémentaires n'étant pas activées.

Etape 104 : deuxième détection : un rayonnement rétrodiffusé $14(\lambda_2)$ est détecté par un photodétecteur élémentaire $20_j$, dans la deuxième bande spectrale $\lambda_2$. Cette détection est synchrone de la deuxième illumination.

Les étapes 101 à 104 sont ensuite répétées, de façon à obtenir une évolution temporelle de chaque rayonnement de détection détecté. Les illuminations des étapes 101 et 103 peuvent être successives et simultanées, en fonction de la capacité des photodétecteurs à séparer les rayonnements rétrodiffusés à chaque longueur d'onde. Les illuminations peuvent être par exemple successives.

Etape 105 : à partir des intensités des premier et deuxième rayonnements de rétrodiffusion détectés lors des différentes étapes 101 et 104, on détermine une réflectance respectivement à la première et à la deuxième longueur d'onde. Par réflectance, on entend une valeur obtenue après traitement d'une intensité d'un rayonnement rétrodiffusé 14. Ce traitement peut être une normalisation de l'intensité détectée, à laquelle on a éventuellement soustrait un terme de bruit parasite, par une intensité $I_0(\lambda_1)$ (respectivement $I_0(\lambda_2)$) du faisceau lumineux incident 12 à la première longueur d'onde $\lambda_1$ ou à la deuxième longueur d'onde $\lambda_2$. On obtient ainsi une réflectance $R(\lambda_i)$ à chaque longueur d'onde correspondant à la distance de rétrodiffusion considérée et à la longueur d'onde $\lambda_i$. La succession des détections synchrones au cours des étapes 101 à 104 fait qu'on peut disposer d'une évolution temporelle $R(\lambda_1,t)$ de la réflectance à la première longueur d'onde et d'une évolution temporelle $R(\lambda_2,t)$ de la réflectance à la deuxième

longeur d'onde. On a schématisé, sur la figure 2B, une évolution temporelle de la réflectance à la première longueur d'onde. Comme décrit en lien avec l'art antérieur, la réflectance fluctue entre des valeurs minimales périodiques formant une ligne de base $min(\lambda_1)$, représentée en pointillés sur la figure 2B, et des valeurs maximales périodiques $max(\lambda_1)$. La fréquence d'occurrence des valeurs maximales (ou minimales) correspond au pouls de l'individu dont le tissu est examiné.

Etape 106 : détermination de valeurs remarquables. A partir de l'évolution temporelle de la réflectance à chaque longueur d'onde, on peut déterminer une minimale *min* et une valeur maximale *max*. L'occurrence de ces valeurs est périodique. On obtient alors $min(\lambda_1)$, $max(\lambda_1)$, $min(\lambda_2)$ et $max(\lambda_2)$.

Etape 107 : estimation : à partir de l'évolution temporelle de la réflectance à chaque longueur d'onde $\lambda_i$, la saturation d'oxygène $Sp(O_2)$, dite pulsatile en raison de la variation périodique des intensités maximales détectées, est estimée selon l'expression :

$$Sp(O_2) = \frac{\mu_{aHb}(\lambda_1) - RR \times \mu_{aHb}(\lambda_2)}{(\mu_{aHb}(\lambda_1) - \mu_{aHbO2}(\lambda_1)) + RR \times (\mu_{aHbO2}(\lambda_2) - \mu_{aHb}(\lambda_2))} \quad (1)$$

Avec

$$RR = \frac{ln\left(\frac{max(\lambda 1)}{min(\lambda 1)}\right)}{ln\left(\frac{max(\lambda 2)}{min(\lambda 2)}\right)} \quad (2)$$

$\mu_{aHb}$ est le coefficient d'absorption de la désoxyhémogobine. $\mu_{aHbO2}$ est le coefficient d'absorption de l'oxyhémogobine.

Etape 108 : estimation de la fréquence cardiaque. A partir de l'intervalle temporel entre deux minimas *min* successifs ou entre deux maximas *max* successifs, on peut estimer une fréquence cardiaque *f* de l'individu duquel le tissu est examiné. Cette étape est optionnelle. La fréquence cardiaque peut également être obtenue par une analyse spectrale des signaux mesurés, ou par une analyse de leur dérivée.

Cette première modalité est basée sur une évolution temporelle du rayonnement rétrodiffusé selon deux longeurs d'onde différentes. Il ne nécessite que l'utilisation d'un seul photodétecteur élémentaire, c'est-à-dire une seule distance de rétrodiffusion. Les inventeurs estiment qu'il est préférable que la distance de rétrodiffusion soit comprise entre 0,5 et 1 cm, et de préférence à 0,7 cm. La figure 3 représente l'évolution de la réflectance mesurée à une longueur d'onde de 660 nm en fonction de la distance de rétrodiffusion $x_j$, exprimée en cm, et cela pour trois types de peaux : une peau claire (III), une peau moyenne (II) et une peau foncée (I). On observe une décroissance rapide de la réflectance en fonction de la rétrodiffusion. Cependant, comme indiqué en lien avec la figure 1B, plus la distance de rétrodiffusion $x_j$ augmente, plus la profondeur maximale $z_j$ des photons formant le rayonnement rétrodiffusé $14_j$ est importante. La proportion des photons du rayonnement rétrodiffusés traversant la vascularisation du tissu peut donc augmenter avec la distance de rétrodiffusion. Une telle proportion peut être quantifiée par un ratio *max/min*. Les inventeurs ont constaté que ce ratio était optimal, à 660 nm ou à 940 nm, lorsque la distance de rétrodiffusion $x_j$ est de 7 mm. Le tableau 1 représente des valeurs du ratio *max/min* obtenus sur différents types de peau, à 660 nm et 940 nm.

Tableau 1

| Couleur peau | claire | moyenne | foncée | claire |
|---|---|---|---|---|
| $\lambda$(nm) | 660 nm | 660 nm | 660 nm | 940 nm |
| *max/min* - $x_j$ = 7 mm | 0.06 | 0.056 | 0.052 | 0.18 |
| *max/min* - $x_j$ = 6 mm | 0.046 | 0.047 | 0.042 | 0.10 |

Aussi, de préférence, le procédé décrit en lien avec la figure 2A est préférentiellement mis en oeuvre en considérant une distance de rétrodiffusion de 7 mm.

Les inventeurs ont également mis en évidence l'influence de la longueur d'onde sur les résultats obtenus. Pour cela, des simulations du trajet des photons constituant le rayonnement rétrodiffusé ont été mises en oeuvre à l'aide d'un code de calcul basé sur une méthode de Monte-Carlo, en faisant varier la longueur d'onde. Les figures 4A, 4B et 4C représentent respectivement les trajets les plus probables des photons de rétrodiffusion, émanant du tissu à une même distance de rétrodiffusion $x$, pour une peau claire, une peau moyenne et une peau sombre. Sur chacune de ces courbes, les ordonnées représentent la profondeur dans le tissu, exprimées en cm, l'ordonnée $y=0$ correspondant à la surface du tissu, et les abscisses représentent la distance de rétrodiffusion, exprimée en cm. Les photons sont injectés à la coordonnée ($x = 0$, $y = 0$). Sur les exemples représentés, la distance de rétrodiffusion est de 7 mm. Les photons représentés émanent du tissu au point de coordonnées ($x = 0,7$, $y = 0$). Le niveau de gris de chaque pixel correspond à une densité de photons. Cela représente une probabilité que le pixel soit situé sur le trajet d'un photon de rétrodiffusion à cette distance de rétrodiffusion dans le tissu. Dans chaque cas de figure, la même quantité de photons a été injectée dans le tissu au niveau d'une zone d'illumination située à l'abscisse 0 cm, et à une profondeur de 0 cm. Sur ces figures, le tissu est illuminé par un faisceau d'illumination à la longueur d'onde de 660 nm. Lors de ces simulations, on a supposé qu'une couche vascularisée s'étend parallèlement à la surface du tissu, à une profondeur de la surface du tissu comprise entre 1.8 et 1.9 mm. Dans cette couche, le coefficient d'absorption est considéré comme représentatif du coefficient d'absorption du sang, et varie entre une valeur correspondant à la diastole et une valeur correspondant à la systole. Les figures 4D, 4E et 4F représentent respectivement les trajets les plus probables des photons de rétrodiffusion pour une peau claire, une peau moyenne et une peau sombre, la longueur d'onde du faisceau d'illumination s'élevant à 740 nm. Lorsque la longueur d'onde est de 740 nm, les photons pénètrent davantage en profondeur dans le tissu. La figure 4G représente un profil de chaque image 4A à 4G en fonction de la profondeur, le profil passant par la ligne verticale en pointillés représentée sur chacune de ces figures. Chaque profil est repéré par la figure à laquelle il se rapporte. On constate que la densité de photons est plus importante entre 0.3 et 1 cm lorsque la première longueur d'onde est 740 nm. Les inventeurs ont également constaté qu'il était plus favorable que la deuxième longueur d'onde soit égale à 870 nm, par rapport à la longueur d'onde de 940 nm couramment utilisée.

Le tableau 2 représente, pour une peau claire, le ratio $\dfrac{\frac{max(\lambda 1)}{min(\lambda 1)}}{\frac{max(\lambda 2)}{min(\lambda 2)}}$ en considérant différents couples de longueurs d'onde $\lambda_1/\lambda_2$

Tableau 2

| $\lambda_1;\lambda_2$ | 660nm ; 940nm | 660nm ; 870nm | 740nm ; 940nm | 740nm ; 870nm |
| --- | --- | --- | --- | --- |
| ratio | 0.33 | 0.5 | 0.44 | 0.66 |

Les inventeurs ont constaté que les mesures de la saturation pulsée selon la première modalité, telle que décrite en lien avec la figure 2A, peuvent conduire à des résultats erronés lorsque le porteur est en mouvement. En effet, un mouvement du porteur peut induire une vibration du dispositif et une fluctuation de la distance d s'étendant entre le dispositif 1 et le tissu examiné 2. Plus généralement, un mouvement du dispositif ou du tissu induit une modification du chemin optique du rayonnement rétrodiffusé, ce qui perturbe la mesure. Or, cette première modalité est basée sur un suivi temporel des réflectances respectivement calculées dans les deux longueurs d'onde. Une fluctuation perturbe ce suivi temporel, conduisant à des erreurs de mesure.

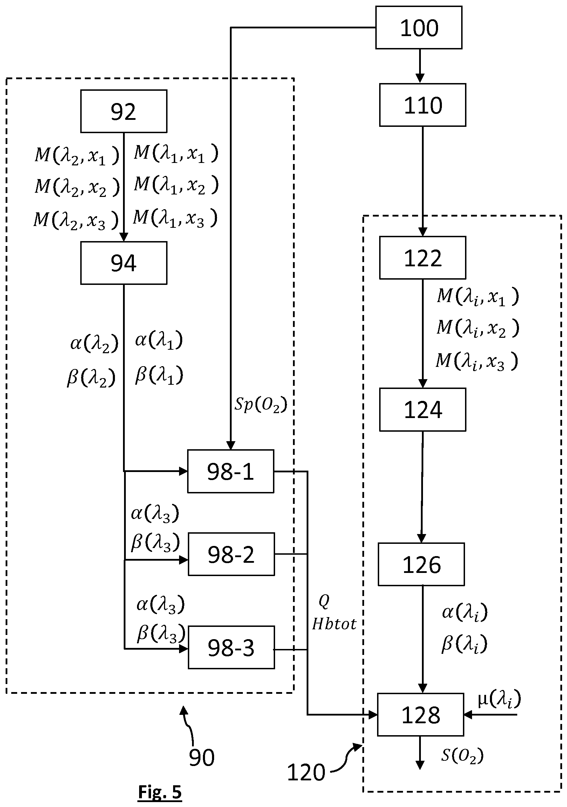

Aussi, le procédé comporte une étape 110 de validation de la teneur en oxygène, et éventuellement de la fréquence cardiaque, déterminées lors de la première modalité, selon l'étape 100. Cette validation est basée sur un mouvement du dispositif 1, mesuré par le capteur de mouvement, ou de valeurs incohérentes obtenues par la première modalité 100. En cas d'invalidation, une deuxième modalité 120 est mise en oeuvre pour procéder à une mesure de la saturation d'oxygène. Cette deuxième modalité est décrite ci-après, en lien avec la figure 5. La deuxième modalité est considérée comme plus robuste à l'égard des mouvements du tissu ou du dispositif, car elle est basée sur une mesure d'une évolution spatiale, et non temporelle, du rayonnement rétrodiffusé selon une même longueur d'onde. Elle repose donc sur une comparaison de l'intensité de signaux rétrodiffusés détectés simultanément. Elle est ainsi moins sensible à une variation, avec le temps, de la position du dispositif par rapport au tissu.

Durant l'estimation de la saturation partielle pulsée $Sp(O_2)$, selon la première modalité, un contrôle de la validité de l'estimation est effectué, au cours de l'étape 110. Ce contrôle mène soit à une validation de l'estimation, auquel cas les étapes 101 à 108 sont réitérées, soit à une invalidation de l'estimation, lorsqu'une défaillance est suspectée. La survenue d'une défaillance peut être détecté par le capteur de mouvement 32. Lorsque l'amplitude et/ou la fréquence du mouvement détecté par le capteur de mouvement dépasse un seuil prédéterminé, on considère que le mouvement subi par le dispositif 1 peut fausser l'estimation. Une estimation de la saturation selon la deuxième modalité 120 est mise en oeuvre. Selon une variante, la survenue d'une défaillance correspond à une fluctuation des rayonnements de rétrodiffusion détectés. Elle peut également correspondre à un seuil ou à une variation de la fréquence cardiaque du porteur mesurée selon l'étape 108, ou à un seuil ou une variation de la teneur en oxygène déterminée lors de l'étape 107.

Les principales étapes de la deuxième modalité 120 sont décrites dans la figure 5. La deuxième modalité utilise une mesure de réflectance selon au moins trois distances de rétrodiffusion $x_j$ différentes les unes des autres, de préférence comprises entre 0.3 cm et 1.5 cm, voire 1 cm. La deuxième modalité 120 est plus robuste que la première modalité à l'égard d'un mouvement. Les principales étapes de cette modalité sont les suivantes :

Etape 122 : Détection de rayonnements rétrodiffusés.

Cette étape comporte l'illumination de l'échantillon dans une longueur d'onde dite d'intérêt, et la détection simultanée d'au moins trois rayonnements rétrodiffusés $14_1(\lambda_i)$, $14_2(\lambda_i)$ et $14_3(\lambda_i)$ selon deux distances de rétrodiffusion différentes $x_1$, $x_2$ et $x_3$. L'intensité mesurée de chaque rayonnement diffusé $14_j(\lambda_i)$, est notée $M(\lambda_i, x_j)$. L'intensité $M(\lambda_i, x_j)$ est avantageusement normalisée par l'intensité du faisceau lumineux $12_i$ émis par la source de lumière $10_i$.

Chaque rayonnement rétrodiffusé émane de la surface du tissu à l'extérieur de la zone élémentaire d'illumination 13, formée par l'intersection du faisceau lumineux incident 12 avec la surface de l'échantillon.

Etape 124 : détermination des réflectances à chaque distance de rétrodiffusion.

Il s'agit de déterminer des réflectances à partir des intensités de chaque signal M($\lambda_i$, $x_1$), M($\lambda_i$, $x_2$) et M($\lambda_i$, $x_3$) détecté lors de l'étape 122.

Les inventeurs ont établi que chaque signal détecté $M(\lambda_i, x_j)$ représente une intégrale de la réflectance selon la distance de rétrodiffusion x, c'est-à-dire la distance par rapport à la zone d'illumination 13. Cela est dû au fait que chaque photodétecteur élémentaire s'étend selon une certaine surface de détection, définissant une distance minimale $x_{j-min}$ et une distance maximale $x_{j-max}$ par rapport à la zone élémentaire d'illumination, avec $x_{j-min} < x_j < x_{j-max}$. Le signal détecté $M(\lambda_i, x_j)$ correspond à une intégrale de la réflectance $R(\lambda_i, x_j)$ entre la distance minimale $x_{j-min}$ et la distance maximale $x_{j-max}$. On peut montrer que la réflectance varie en fonction de la distance de rétrodiffusion selon une loi générale de type :

$$R(\lambda_i, x) = \frac{\beta(\lambda_i)}{x^2} e^{-\alpha(\lambda_i)x} \quad (4)$$

- où $\beta(\lambda_i)$ et $\alpha(\lambda_i)$ sont des coefficients dépendant de $\lambda_i$ ;
- x représente la distance de rétrodiffusion, c'est-à-dire la distance à laquelle émane le signal de rétrodiffusion par rapport à la zone élémentaire d'illumination 13.
-
$$\beta(\lambda) = \frac{6z_0}{4\pi} \cdot \mu_{\text{eff}}(\lambda) \quad (4')$$

et

$$\alpha(\lambda) = \mu_{\text{eff}}(\lambda) \quad (4''),$$

avec $z_0 \approx \dfrac{1}{\mu_s'}$ et $\mu_{\text{eff}} \approx \sqrt{3\mu_a \mu_s'}$, en se basant sur une hypothèse $\mu_a << \mu_s'$,

Les inventeurs ont vérifié que le signal détecté par les photodétecteurs élémentaires varie, en fonction de la distance x, selon une expression de type $\dfrac{a(\lambda_i)}{x} e^{-b(\lambda_i)x}$, $a(\lambda_i)$ et $b(\lambda_i)$ étant des coefficients dépendants de $\lambda_i$.

Ainsi, l'intensité M($\lambda_i$, $x_j$) du rayonnement rétrodiffusé $14_j$ mesuré par les détecteurs, aux différentes distances de rétrodiffusion $x_j$, est dérivée, par rapport à x de façon à obtenir une grandeur représentative de la réflectance $R_{j,j+1}$ à une distance de rétrodiffusion $x_{j,j+1}$ deux distances de rétrodiffusions $x_j$ et $x_{j+1}$ consécutives.

Les distances de rétrodiffusions étant discrètes, la dérivée est approximée par un taux de variation, de telle sorte qu'on établit les calculs suivants :

$$R_{1,2} = \frac{\partial M}{\partial x}(\lambda_i, x_{1,2}) = \frac{M(\lambda_i, x_1) - M(\lambda_i, x_2)}{(x_1 - x_2)} \quad (5)$$

$$R_{2,3} = \frac{\partial M}{\partial x}(\lambda_i, x_{2,3}) = \frac{M(\lambda_i, x_2) - M(\lambda_i, x_3)}{(x_2 - x_3)} \quad (5')$$

Etape 126 : calcul des paramètres $\alpha(\lambda_i)$ et $\beta(\lambda_i)$.

En combinant les expressions (4), (4'), (4") avec les expressions (5) et (5'), on peut montrer que

.

$$\alpha(\lambda_i) = 2 \frac{\ln\big(M(\lambda_i,x_1)-M(\lambda_i,x_2)\big)-\ln\big(M(\lambda_i,x_2)-M(\lambda_i,x_3)\big)-\ln(x_1-x_2)+\ln(x_2-x_3)-\ln(x_2+x_3)^2+\ln(x_1+x_2)^2}{(x_1-x_3)}$$

(6)

et

$$\beta(\lambda_i) = \frac{M(\lambda_i,x_1)-M(\lambda_i,x_2)}{(x_1-x_2)} \times \frac{(x_1+x_2)^2}{2} \times e^{\alpha(\lambda_i)\frac{(x_1+x_2)}{2}} \ (6')$$

D'une façon générale, les étapes 124 et 126 consistent à combiner les intensités des rayonnements rétrodiffusés $M(\lambda_i,x_j)$, à la longueur d'onde $\lambda_i$, selon au moins trois différentes distances de rétrodiffusion $x_j$ pour obtenir les coefficients $\alpha(\lambda_i)$ et $\beta(\lambda_i)$. Ces coefficients permettent d'exprimer la réflectance en fonction de la distance de rétrodiffusion. Les intensités des rayonnements rétrodiffusés sont soustraites deux à deux ; il en est de même pour les distances de rétrodiffusion.

Les coefficients $\alpha(\lambda_i)$ et $\beta(\lambda_i)$ peuvent également être obtenus par un ajustement, en considérant l'équation (4), à partir de mesures de réflectance $R(\lambda_i, x_j)$ à différentes distances de rétrodiffusion $x_j$.

Etape 128 : détermination de la saturation $S(O_2)$.

Le coefficient d'absorption du tissu $\mu_a(\lambda_i)$ peut s'exprimer de la façon suivante :

$$\mu_a(\lambda_i) = [Hbtot]\big(S\mu_{aHbO2}(\lambda_i) + (1-S)\mu_{aHb}(\lambda_i)\big) + [1-Hbtot]\mu_{aother}(\lambda_i) = \frac{2\pi.\alpha(\lambda_i)\beta(\lambda_i)}{9} \ (7)$$

Il vient alors :

$$[Hbtot] = \frac{\frac{2\pi.\alpha(\lambda)\beta(\lambda)}{9}-\mu_{aother}(\lambda_i)}{\big(S(O_2)\cdot\delta\mu(\lambda_i)+\mu_{aHb}(\lambda_i)-\mu_{aother}(\lambda_i)\big)} \ (8)$$

avec

$$\delta\mu(\lambda_i) = \mu_{aHbO2}(\lambda_i) - \mu_{aHb}(\lambda_i) \ (9)$$

ce qui donne

$$S(O_2) = \frac{\frac{2\pi.\alpha(\lambda_i)\beta(\lambda_i)}{9}-\mu_{aHb}(\lambda_i)[Hbtot]-(1-[Hbtot])\mu_{aother}(\lambda_i)}{\delta\mu(\lambda_i).[Hbtot]} \ (10)$$

Les inconnues de cette équation sont $[Hbtot]$ et $\mu_{aother}(\lambda_i)$. La grandeur à mesurer est $S(O_2)$. Les autres termes sont :

- soit connus : il s'agit de $\mu_{aHb}(\lambda_i)$, $\delta\mu(\lambda_i)$;
- soit déterminés à partir de la mesure: il s'agit de $\alpha(\lambda_i)$ et $\beta(\lambda_i)$, déterminés selon les expressions (6) et (7), au cours de l'étape 126.

Les inconnues $[Hbtot]$ et $\mu_{aother}(\lambda_i)$ dépendent de l'individu, ainsi que de la position du dispositif de mesure sur ce dernier. $[Hbtot]$ est une concentration en hémoglobine du tissu examiné, et $\mu_{aother}(\lambda_i)$ représente une atténuation du tissu par des composants de ce dernier autres que l'hémoglobine. L'absorption $\mu_a(\lambda_i)$ dans le tissu examiné est essentiellement gouvernée par l'oxyhémoglobine, la déoxyhémoglobine, ainsi que par d'autres composants du tissu désignés par le terme *other*. L'absorption de ces autres composants peut être quantifiée par le coefficient d'absorption global noté $\mu_{a\text{-}other}(\lambda_i)$. Le coefficient d'absorption global $\mu_{a\text{-}other}(\lambda_i)$ dépend d'un individu à un autre, mais on trouve, dans la littérature, des expressions permettant d'obtenir une dépendance du coefficient d'absorption lié à ces composants, noté $\mu_{a\text{-other}}(\lambda_i)$ en fonction de la longueur d'onde $\lambda_i$.

Par exemple la relation

$$\mu_{a-other}(\lambda_i) = 7.84 \times 10^8 \times \lambda_i^{-3.255} \quad (11)$$

est donnée par Meglinski dans Meglinski, I. et al "Computer simulation of the skin reflectance spectra computer methods and programs in biomedecine", Elsevier, 2003, 70, 179-186.

Par ailleurs, la relation

$$\mu_{a-other}(\lambda_i) = 0.244 + 85.3e^{\frac{\lambda_i-154}{66.2}} \quad (11')$$

est donnée dans http://omlc.org.news/jan98/skinoptics.html

Ainsi, $\mu_{a\text{-other}}(\lambda_i)$ suit une fonction connue $\mu(\lambda_i)$, et disponible dans la littérature. Afin de tenir compte d'une variabilité entre individus, les inventeurs considèrent que :

$$\mu_{a-other}(\lambda_i) = Q \times \mu(\lambda_i) \quad (11)$$

$\mu(\lambda_i)$ est une fonction décrivant l'évolution de $\mu_{a\text{-other}}$ en fonction de $\lambda_i$

Q est un facteur de variabilité dépendant de chaque individu, ainsi que de la partie de tissu examiné. Q dépend notamment de la concentration en mélanine, en eau, en bilirubine, ou en graisse du tissu observé. Q est une grandeur scalaire, qui est considérée comme indépendante de la longueur d'onde, ce qui permet de simplifier l'équation (10), en prenant en compte l'expression (11) :

$$S(O_2) = \frac{\frac{2\pi.\alpha(\lambda_i)\beta(\lambda_i)}{9} - \mu_{aHb}(\lambda_i)[Hbtot] - (1-[Hbtot])Q \times \mu(\lambda_i)}{\delta\mu(\lambda_i).[Hbtot]} \quad (12)$$

Les inconnues $Q$ et *[Hbtot]* forment respectivement un premier terme de calibration et un deuxième terme de calibration ; ils sont déterminés au cours d'une phase préalable de calibration 90, dont les étapes sont décrites ci-dessous.

Etape 92 : mesures du signal rétrodiffusé à deux longueurs d'onde

La phase de calibration comporte une mesure de signaux rétrodiffusés, à au moins deux longueurs d'onde $\lambda_1$ et $\lambda_2$, qui sont, dans cet exemple, les longueurs d'onde utilisées lors de l'application de la première modalité. La mesure est réalisée aux trois distances de rétrodiffusion $x_1$, $x_2$ et $x_3$. On mesure alors, à chaque longueur d'onde, les intensités $M(\lambda_i, x_1)$, $M(\lambda_i, x_2)$ et $M(\lambda_i, x_3)$ de chaque rayonnement rétrodiffusé.

Etape 94 : Comparaison des signaux mesurés $M(\lambda_i, x_1)$, $M(\lambda_i, x_2)$ et $M(\lambda_i, x_3)$ et des distances de rétrodiffusion, pour calculer les coefficients $\alpha(\lambda_i)$ et $\beta(\lambda_i)$.

Il s'agit d'appliquer les équations (5) et (5') de façon à calculer les coefficients $\alpha(\lambda_i)$ et $\beta(\lambda_i)$ selon les expressions (6) et (6').

Etape 98: Calcul des inconnues.

Les inconnues à déterminer sont *[Hbtot]* et $Q$, la fonction $\mu(\lambda_i)$ étant déterminé selon une expression connue, par exemple selon les équations (11) ou (11'). Plusieurs variantes permettent d'obtenir ces inconnues.

Selon une première variante 98.1, on utilise une mesure validée de la saturation pulsée d'oxygène $Sp(O_2)$, mesurée à l'aide de la première modalité, à un instant, dit instant de calibration. De cette façon, à cet instant, $S(O_2) = Sp(O_2)$. L'expression (12), exprimée aux longueurs d'onde $\lambda_1$ et $\lambda_2$, forment un système à deux équations et à deux inconnues, ce qui permet de déterminer *[Hbtot]* et $Q$. Ces paramètres dépendent de l'individu et de la position du dispositif sur l'individu. Suite à la calibration, l'expression (12) permet de déterminer $S(O_2)$ selon la deuxième modalité, en utilisant une seule longueur d'onde $\lambda_1$ ou $\lambda_2$ ou les deux longueurs d'onde. Selon cette variante, la première modalité est mise en oeuvre et la deuxième modalité est calibrée à l'aide d'une mesure $Sp(O_2)$ issue de la première modalité. L'intérêt de cette variante est que la mesure $Sp(O_2)$ est obtenue par une méthode éprouvée.

Selon une deuxième variante 98-2, on utilise une troisième longueur d'onde $\lambda_3$, correspondant à une longueur d'onde isobestique du spectre d'absorption de l'hémoglobine. A une telle longueur d'onde, l'absorption de l'oxyhémoglobine est identique à l'absorption de la désoxyhémoglobine : $\delta\mu(\lambda_3) = 0$. Cette longueur d'onde peut être égale à 805 nm, mais il est possible d'utiliser une autre longueur d'onde isobestique, par exemple 507 nm, 522 nm, 549 nm, 569 nm ou 586 nm. A la longueur d'onde isobestique $\lambda_3$, $\delta\mu(\lambda_3) = 0$, ce qui permet d'obtenir l'expression :

$$[Hbtot] = \frac{\frac{2\pi.\alpha(\lambda_3)\beta(\lambda_3)}{9} - \mu_{aother}(\lambda_3)}{(\mu_{aHb}(\lambda_3) - \mu_{aother}(\lambda_3))} = \frac{\frac{2\pi.\alpha(\lambda_3)\beta(\lambda_3)}{9} - Q\times\mu(\lambda_3)}{(\mu_{aHb}(\lambda_3) - Q\times\mu(\lambda_3))} \quad (14)$$

Appliquée aux deux longueurs d'onde $\lambda_1$ et $\lambda_2$, l'expression (12) permet d'écrire :

$$\frac{\frac{2\pi.\alpha(\lambda_1)\beta(\lambda_1)}{9} - \mu_{aHb}(\lambda_1)[Hbtot] - (1-[Hbtot])Q\times\mu(\lambda_1)}{\delta\mu(\lambda_1).[Hbtot]} = \frac{\frac{2\pi.\alpha(\lambda_2)\beta(\lambda_2)}{9} - \mu_{aHb}(\lambda_2)[Hbtot] - (1-[Hbtot])Q\times\mu(\lambda_2)}{\delta\mu(\lambda_2).[Hbtot]}$$

$$(15).$$

On dispose ainsi de deux équations (14) et (15) dont les inconnues sont Q et *[Hbtot]*.

Selon une troisième variante 98-3, on utilise une troisième longueur d'onde $\lambda_3$, qui n'est pas nécessairement isobestique. Appliquée aux trois longueurs d'onde, l'expression (12) devient :

$$\frac{\frac{2\pi.\alpha(\lambda_1)\beta(\lambda_1)}{9} - \mu_{aHb}(\lambda_1)[Hbtot] - (1-[Hbtot])Q\times\mu(\lambda_1)}{\delta\mu(\lambda_1).[Hbtot]} = \frac{\frac{2\pi.\alpha(\lambda_2)\beta(\lambda_2)}{9} - \mu_{aHb}(\lambda_2)[Hbtot] - (1-[Hbtot])Q\times\mu(\lambda_2)}{\delta\mu(\lambda_2).[Hbtot]}$$

$$(15)$$

et

$$\frac{\frac{2\pi.\alpha(\lambda_3)\beta(\lambda_3)}{9} - \mu_{aHb}(\lambda_3)[Hbtot] - (1-[Hbtot])Q\times\mu(\lambda_3)}{\delta\mu(\lambda_3).[Hbtot]} = \frac{\frac{2\pi.\alpha(\lambda_2)\beta(\lambda_2)}{9} - \mu_{aHb}(\lambda_2)[Hbtot] - (1-[Hbtot])Q\times\mu(\lambda_2)}{\delta\mu(\lambda_2).[Hbtot]}$$

$$(16)$$

On dispose ainsi de deux équations (15) et (16) dont les inconnues sont *Q* et *[Hbtot]*.

L'étape 98 permet de calculer les inconnues *Q* et *[Hbtot]* permettant la détermination de la saturation d'oxygène en mettant en oeuvre l'étape 128.

On note que la deuxième modalité peut être mise en oeuvre de façon complémentaire ou indépendante de la première modalité. Lorsque les deux modalités sont combinées, la saturation pulsée d'oxygène déterminée à partir de la première modalité permet de calibrer la deuxième modalité (première variante 98-1 de la calibration). Dans ce cas, lors de la calibration de la deuxième modalité, le tissu peut être illuminé selon une des deux longueurs d'onde mises en oeuvre par la première modalité.

La première modalité repose essentiellement sur une évolution temporelle d'un rayonnement rétrodiffusé par l'échantillon, à une distance de rétrodiffusion. Elle est donc sensible à des mouvements du tissu ou du dispositif, ces derniers étant susceptibles de modifier l'évolution temporelle, et d'entraîner une estimation erronée de la saturation d'oxygène. Cela apparaît notamment lorsque la position du dispositif de mesure, par rapport au tissu, varie selon ou plus rapidement que la fréquence cardiaque. Dans un tel cas, les valeurs min et max obtenues selon la première modalité subissent une fluctuation non maîtrisée, pouvant conduire à une erreur d'estimation.

La deuxième modalité est basée sur une mesure d'une évolution spatiale du rayonnement rétrodiffusé par l'échantillon. Elle suppose la prise en compte d'au moins trois distances de rétrodiffusion, auxquelles le rayonnement rétrodiffusé est mesuré simultanément, de façon synchrone à l'illumination. La deuxième modalité est donc moins sensible aux fluctuations temporelles de la position du dispositif par rapport au tissu, puisque les données nécessaires à la détermination de la saturation d'oxygène sont acquises simultanément. La deuxième modalité est donc adaptée aux situations dans lesquelles la position du dispositif de mesure est susceptible de varier rapidement. Elle convient donc particulièrement à l'estimation de la saturation en oxygène chez un sujet en mouvement.

Lorsque la deuxième modalité est mise en oeuvre de façon indépendante de la première modalité, sa calibration peut nécessiter le recours à trois longueurs d'onde différentes. La troisième longueur d'onde peut être avantageusement une longueur d'onde isobestique, ou située dans une bande spectrale s'étendant autour d'une longueur d'onde isobestique. Dans ce cas, la calibration de la mesure est réalisée selon la deuxième variante 98-2 de la calibration. Mais la troisième longueur d'onde peut être différente d'une longueur d'onde isobestique, auquel cas la deuxième modalité est calibrée selon la troisième variante (étape 98-3) de la calibration.

## Revendications

**1.** Procédé d'estimation d'une teneur en oxygène dans un tissu corporel vivant (2), comportant les étapes suivantes:

a) illumination d'une surface (2s) du tissu par un faisceau lumineux incident (12i), selon une longueur d'onde d'intérêt ($\lambda_i$), de façon à former une zone élémentaire d'illumination (13, $13_i$), correspondant à la partie de la surface éclairée par le faisceau lumineux incident;

b) au cours de l'illumination, détection simultanée d'un rayonnement rétrodiffusé ($14_1$, $14_2$, $14_3$) émanant de la surface de l'échantillon au niveau d'au moins trois zones élémentaires de détection ($15_1$, $15_2$, $15_3$), chaque zone élémentaire de détection respectivement s'étendant à une distance ($x_1$, $x_2$, $x_3$), dite distance de rétrodiffusion, de la zone élémentaire d'illumination (13, $13_i$), chaque distance de rétrodiffusion étant différente l'une de l'autre et inférieure à 15 mm ;

le procédé étant **caractérisé en ce qu'**il comporte également :

c) comparaisons, deux à deux, des intensités des rayonnements rétrodiffusés détectés, ($M(\lambda_i, x_1)$; $M(\lambda_i, x_2)$, $M(\lambda_i, x_3)$), éventuellement normalisées par une intensité du faisceau lumineux incident ($12_i$), ainsi que des distances ($x_1$, $x_2$, $x_3$) de rétrodiffusion, pour déterminer deux coefficients ($\alpha(\lambda i)$, $\beta(\lambda i)$) ;

d) prise en compte d'un premier terme de calibration (*Hbtot*), représentant une quantité d'hémoglobine dans le tissu observé, d'un deuxième terme de calibration (Q), caractérisant une absorption du tissu par des composants du tissu autres que l'hémoglobine, le premier terme de calibration et le deuxième terme de calibration étant déterminés au cours d'une phase de calibration ;

e) estimation d'une teneur en oxygène ($S(O_2)$) du tissu, à partir des comparaisons effectuées lors de l'étape c) et des termes de calibration pris en compte lors de l'étape d, l'étape e) comportant une application d'une expression (12) reliant la teneur en oxygène ($S(O_2)$) aux termes de calibration pris en compte lors de l'étape d) ainsi qu'aux coefficients obtenus lors de l'étape c), l'expression étant basée sur des valeurs connues de coefficients d'absorption de l'oxyhémoglobine ($\mu_{aHbO2}(\lambda_i)$) et de la désoxyhémoglobine ($\mu_{aHb}(\lambda_i)$) à la longueur d'onde d'intérêt,

le procédé étant tel que les termes de calibration pris en compte lors de l'étape d) sont déterminés :

- soit au cours d'une phase préalable de calibration, au cours de laquelle on dispose d'une estimation de la teneur en oxygène du tissu ($Sp(O_2)$), la phase de calibration comportant les étapes suivantes :

cal-i) mise en oeuvre des étapes a) à c), à deux longueurs d'onde différentes ($\lambda_1$, $\lambda_2$);
cal-ii) utilisation de l'expression appliquée lors de l'étape e) à chaque longueur d'onde, en considérant la teneur en oxygène estimée ;

- soit au cours d'une phase préalable de calibration comportant les étapes suivantes :

cal-i) mise en oeuvre des étapes a) à c), à trois longueurs d'onde différentes ($\lambda_1$, $\lambda_2$, $\lambda_3$), une desdites longueurs d'onde étant une longueur d'onde dite isobestique, à laquelle le coefficient d'absorption de l'oxyhémoglobine est considéré comme égal au coefficient d'absorption de la déoxyhémoglobine ;
cal-ii) à partir de l'expression appliquée lors de l'étape e), à chaque longueur d'onde, obtention des termes de calibration.

- soit au cours d'une phase préalable de calibration comportant les étapes suivantes :

cal-i) mise en oeuvre des étapes a) à c), à trois longueurs d'onde différentes ($\lambda_1$, $\lambda_2$, $\lambda_3$);
cal-ii) à partir de l'expression appliquée lors de l'étape e), à chaque longueur d'onde, obtention des termes de calibration;

les étapes c) à e) étant mises en oeuvre à l'aide d'un processeur (30).

2. Procédé selon la revendication 1, dans lequel lors de l'étape b), chaque distance de rétrodiffusion ($x_1$, $x_2$, $x_3$) est supérieure à 0.3 cm.

3. Procédé selon l'une quelconque des revendications précédentes dans lequel l'étape c) comporte les sous-étapes suivantes :

ci) soustraction des intensités de rayonnements de rétrodiffusion détectés à deux distances de rétrodiffusion différentes ($M(\lambda_i, x_1)$ - $M(\lambda_i, x_2)$, $M(\lambda_i, x_2)$ - $M(\lambda_i, x_3)$);
cii) calcul d'un logarithme de ladite soustraction ($\ln(M(\lambda_i, x_1) - M(\lambda_i, x_2))$, $\ln(M(\lambda_i, x_2) - M(\lambda_i, x_3))$);
ciii) soustraction de deux distances de rétrodiffusion différentes (($x_1$ - $x_3$), ($x_1$ - $x_2$)).

4. Procédé bimodal de détermination d'une teneur en oxygène dans un tissu corporel vivant (2), le procédé comportant une première détermination d'une teneur en oxygène selon une première modalité, selon les étapes suivantes

la) illumination d'une surface (2s) du tissu par un faisceau lumineux incident (12i), selon première longueur d'onde ($\lambda_1$) et une deuxième longueur d'onde ($\lambda_2$), de façon à former, lors de chaque illumination, une zone élémentaire d'illumination (13$_i$), correspondant à la partie de la surface éclairée par le faisceau lumineux incident;
1b) au cours de chaque illumination, détection d'un rayonnement rétrodiffusé émanant de la surface de l'échantillon à une même distance ($x_j$), dite distance de rétrodiffusion, la distance de rétrodiffusion étant inférieure à 15 mm, de façon à obtenir un rayonnement rétrodiffusé détecté respectivement à la première longueur d'onde ($\lambda_1$) et à la deuxième longueur d'onde ($\lambda_2$);
1c) à partir de chaque rayonnement rétrodiffusé ainsi détecté, calcul d'une réflectance à la première et à la deuxième longueur d'onde ($R(\lambda_1)$ $R(\lambda_2)$) ;
1d) réitération des trois étapes 1a) à 1c) de façon à obtenir une évolution temporelle de la réflectance du tissu ($R(\lambda_1, t)$ $R(\lambda_2, t)$) à la première et à la deuxième longueur d'onde ;
le) détermination d'une valeur minimale périodique ($min(\lambda_1)$, $min(\lambda_2)$) et d'une valeur maximale périodique ($max(\lambda_1)$, $max(\lambda_2)$) de l'évolution temporelle de la réflectance ($R(\lambda_1, t)$ $R(\lambda_2, t)$), obtenue lors de l'étape 1d) à chaque longueur d'onde ;
1f) calcul d'un ratio $\left(\dfrac{\max(\lambda 1)}{\min(\lambda 1)}, \dfrac{\max(\lambda 2)}{\min(\lambda 1)}\right)$, à chaque longueur d'onde, de la valeur maximale périodique et de la valeur minimale périodique déterminées lors de l'étape 1e) ;
1g) détermination de la teneur en oxygène ($Sp(O_2)$) du tissu à partir des ratios calculés lors de l'étape 1f) à chaque longueur d'onde ;

le procédé étant **caractérisé en ce qu'**il comporte une étape de validation ou d'invalidation de la teneur en oxygène ($Sp(O_2)$) déterminée lors de l'étape 1g), et **en ce qu'**en cas d'invalidation, la teneur en oxygène est déterminée selon une deuxième modalité, la deuxième modalité étant mise en oeuvre selon un procédé objet de l'une quelconque des revendications 1 à 3.

5. Procédé selon la revendication 4, dans lequel la première longueur d'onde ($\lambda_1$) est comprise entre 730 et 750 nm, et dans lequel la deuxième longueur d'onde ($\lambda_2$) est comprise entre 860 et 880 nm.

6. Procédé selon l'une quelconque des revendications 4 ou 5, dans lequel lors de l'étape 1b), la distance de rétrodiffusion ($x_j$) est comprise entre 6 mm et 8 mm.

7. Procédé selon l'une quelconque des revendications 4 à 6, dans lequel la longueur d'onde d'intérêt ($\lambda_i$) utilisée lors de la deuxième modalité est soit la première longueur d'onde ($\lambda_1$), soit la deuxième longueur d'onde ($\lambda_2$).

8. Procédé selon l'une quelconque des revendications 4 à 7, dans lequel :

- l'étape 1a) est mise en oeuvre à l'aide d'une source de lumière (11) ;
- l'étape 1b) est mise en oeuvre à l'aide d'un photodétecteur élémentaire (20$_j$);

la source de lumière et/ou le photodétecteur élémentaire étant solidaires d'un capteur de mouvement (32), de telle sorte que lorsqu'un mouvement significatif est détecté par le capteur de mouvement, la première modalité est invalidée.

9. Procédé selon l'une quelconque des revendications 4 à 8, comportant également une étape de mesure d'une fréquence cardiaque (f) relative au tissu corporel vivant, de telle sorte que lorsque la fréquence cardiaque, ou lorsqu'une variation de la fréquence cardiaque, franchit un certain seuil, la première modalité est invalidée.

10. Dispositif d'oxymétrie (1), apte à être appliqué contre le corps d'un individu ou d'un animal, comportant :

- une première source de lumière ($10_1$) apte à émettre une lumière dans une longueur d'onde comprise entre 600 nm et 800 nm ;
- une deuxième source de lumière ($10_2$) apte à émettre une lumière dans une longueur d'onde comprise entre 800 nm et 1000 nm ;
- au moins trois photodétecteurs élémentaires ($20_1$, $20_2$, $20_3$), s'étendant à trois distances ($x_1$, $x_2$, $x_3$), dites distances de rétrodiffusion, d'une desdites sources de lumière, les distances de rétrodiffusion étant inférieures à 1.5 cm ;
- un processeur, apte à mettre en oeuvre les étapes c) à e) d'un procédé selon l'une quelconque des revendications 1 à 3, à partir de rayonnements de rétrodiffusion détecté par chaque photodétecteur élémentaire durant l'activation de la première source de lumière ou de la deuxième source de lumière ;
- le processeur étant également weist apte à mettre en oeuvre les étapes 1c) à 1g) d'un procédé selon l'une quelconque des revendications 4 à 9, à partir d'un rayonnement rétrodiffusé détecté par un photodétecteur élémentaire ($20_1$, $20_2$, $20_3$) durant l'activation de la première source de lumière et de la deuxième source de lumière.

11. Dispositif selon la revendication 10, comportant un capteur de mouvement (32) configuré pour détecter un mouvement du dispositif.

12. Dispositif selon la revendication 10 ou la revendication 11, dans lequel les distances de rétrodiffusion ($x_1$, $x_2$, $x_3$) sont respectivement comprises entre 3 mm et 10 mm.

## Patentansprüche

1. Verfahren zur Schätzung eines Sauerstoffgehalts in einem lebenden Körpergewebe (2), das die folgenden Schritte aufweist:

a) Beleuchtung einer Fläche (2s) des Gewebes mit einem einfallenden Lichtstrahl (12i) gemäß einer interessierenden Wellenlänge ($\lambda_i$), um eine elementare Beleuchtungszone (13, 13i) zu bilden, die dem vom einfallenden Lichtstrahl beleuchteten Teil der Fläche entspricht;

b) während der Beleuchtung gleichzeitige Erfassung einer rückgestreuten Strahlung ($14_1$, $14_2$, $14_3$), die von der Fläche der Probe im Bereich von mindestens drei elementaren Erfassungszonen ($15_1$, $15_2$, $15_3$) ausgeht, wobei jede elementare Erfassungszone sich in einem Abstand ($x_1$, $x_2$, $x_3$), Rückstreuabstand genannt, von der elementaren Beleuchtungszone (13, 13i) erstreckt, wobei jeder Rückstreuabstand sich voneinander unterscheidet und geringer als 15 mm ist;

wobei das Verfahren **dadurch gekennzeichnet ist, dass** es ebenfalls aufweist:

c) paarweise Vergleiche der Stärken der erfassten rückgestreuten Strahlungen, ($M(\lambda_i,x_1)$; $M(\lambda_i,x_2)$, $M(\lambda_i,x_3)$), ggf. normiert durch eine Stärke des einfallenden Lichtstrahls (12;), sowie der Rückstreuabstände ($x_1$, $x_2$, $x_3$), um zwei Koeffizienten ($\alpha(\lambda i)$, $\beta(\lambda i)$) zu bestimmen;

d) Berücksichtigung eines ersten Kalibrierungsterms (Hbtot), der eine Hämoglobinmenge im betrachteten Gewebe darstellt, eines zweiten Kalibrierungsterms (Q), der eine Absorption des Gewebes durch Bestandteile des Gewebes anders als Hämoglobin kennzeichnet, wobei der erste Kalibrierungsterm und der zweite Kalibrierungsterm während einer Kalibrierungsphase bestimmt werden;

e) Schätzung eines Sauerstoffgehalts ($S(O_2)$) des Gewebes, ausgehend von den im Schritt c) durchgeführten Vergleichen und den im Schritt d) berücksichtigten Kalibrierungstermen, wobei der Schritt e) eine Anwendung eines Ausdrucks (12) aufweist, der den Sauerstoffgehalt ($S(O_2)$) mit den im Schritt d) berücksichtigten Kalibrierungstermen sowie mit den im Schritt c) erhaltenen Koeffizienten verbindet, wobei der Ausdruck auf bekannten Werten von Absorptionskoeffizienten des Oxyhämoglobins ($\mu_{\alpha HbO2}(\lambda_i)$) und des Desoxyhämoglobins ($\mu_{\alpha Hb}(\lambda_i)$) bei der interessierenden Wellenlänge basiert, wobei das Verfahren so ist, dass die im Schritt d) berücksichtigten Kalibrierungsterme bestimmt werden:

- entweder während einer vorhergehenden Kalibrierungsphase, während der man über eine Schätzung des Sauerstoffgehalts des Gewebes ($Sp(O_2)$) verfügt, wobei die Kalibrierungsphase die folgenden Schritte aufweist:

cal-i) Durchführung der Schritte a) bis c) bei zwei unterschiedlichen Wellenlängen ($\lambda_1$, $\lambda_2$);
cal-ii) Verwendung des im Schritt e) angewendeten Ausdrucks bei jeder Wellenlänge, unter Berücksichtigung des geschätzten Sauerstoffgehalts;

- oder während einer vorhergehenden Kalibrierungsphase, die die folgenden Schritte aufweist:

cal-i) Durchführung der Schritte a) bis c) mit drei unterschiedlichen Wellenlängen ($\lambda_1$, $\lambda_2$, $\lambda_3$), wobei eine der Wellenlängen eine so genannte isobestische Wellenlänge ist, bei der der Absorptionskoeffizient des Oxyhämoglobins als gleich dem Absorptionskoeffizienten des Deoxyhämoglobins angesehen wird;
cal-ii) ausgehend vom im Schritt e) angewendeten Ausdruck bei jeder Wellenlänge Erhalt der Kalibrierungsterme;

- oder während einer vorhergehenden Kalibrierungsphase, die die folgenden Schritte aufweist:

cal-i) Durchführung der Schritte a) bis c) mit drei unterschiedlichen Wellenlängen ($\lambda_1$, $\lambda_2$, $\lambda_3$);
cal-ii) ausgehend vom im Schritt e) auf jede Wellenlänge angewendeten Ausdruck Erhalt der Kalibrierungsterme;
wobei die Schritte c) bis e) mit Hilfe eines Prozessors (30) durchgeführt werden.

2. Verfahren nach Anspruch 1, wobei im Schritt b) jeder Rückstreuabstand ($x_1$, $x_2$, $x_3$) größer ist als 0,3 cm.

3. Verfahren nach einem der vorhergehenden Ansprüche, wobei der Schritt c) die folgenden Teilschritte aufweist:

ci) Subtraktion der Stärken von Rückstreustrahlungen, die in zwei unterschiedlichen Rückstreuabständen ($M(\lambda_i,x_1)$-$M(\lambda_i,x_2)$, $M(\lambda_i,x_2)$-$M(\lambda_i,x_3)$) erfasst wurden;
cii) Berechnung eines Logarithmus der Subtraktion ($\ln(M)(\lambda_i,x_1)$-$(M(\lambda_i,x_2))$, $\ln(M(\lambda_i,x_2)$-$M(\lambda_i,x_3)))$ ;
ciii) Subtraktion von zwei unterschiedlichen Rückstreuabständen (($x_1$ - $x_3$), ($X1$ - $X2$)).

4. Bimodales Verfahren zur Bestimmung eines Sauerstoffgehalts in einem lebenden Körpergewebe (2), wobei das Verfahren eine erste Bestimmung eines Sauerstoffgehalts gemäß einer ersten Modalität gemäß den folgenden Schritten aufweist

1a) Beleuchtung einer Fläche (2s) des Gewebes mit einem einfallenden Lichtstrahl (12i) gemäß einer ersten Wellenlänge ($\lambda_1$) und einer zweiten Wellenlänge ($\lambda_2$), um bei jeder Beleuchtung eine elementare Beleuchtungszone (13i) zu bilden, die dem vom einfallenden Lichtstrahl beleuchteten Teil der Fläche entspricht;
1b) während jeder Beleuchtung Erfassung einer rückgestreuten Strahlung, die von der Fläche der Probe in einem gleichen Abstand ($x_j$), Rückstreuabstand genannt, ausgeht, wobei der Rückstreuabstand kleiner als 15 mm ist, um eine rückgestreute Strahlung zu erhalten, die mit der ersten Wellenlänge ($\lambda_1$) bzw. der zweiten Wellenlänge ($\lambda_2$) erfasst wird;
1c) ausgehend von jeder so erfassten rückgestreuten Strahlung Berechnung einer Reflektanz mit der ersten und der zweiten Wellenlänge ($R(\lambda_1)$ $R(\lambda_2)$);
1d) Wiederholung der drei Schritte 1a) bis 1c), um eine zeitliche Entwicklung der Reflektanz des Gewebes ($R(\lambda_i,t)$ $R(\lambda_2,t)$) mit der ersten und der zweiten Wellenlänge zu erhalten;
1e) Bestimmung eines periodischen minimalen Werts ($\min(\lambda_1)$), $\min(\lambda_2)$) und eines periodischen maximalen Werts ($\max(\lambda_1)$), $\max(\lambda_2)$) der zeitlichen Entwicklung der im Schritt 1d) erhaltenen Reflektanz ($R(\lambda_1,t)$ $R(\lambda_2,t)$) bei jeder Wellenlänge;

1f) Berechnung eines Verhältnisses $\left( \frac{max(\lambda 1)}{min(\lambda 1)}, \frac{max(\lambda 2)}{min(\lambda 1)} \right)$ bei jeder Wellenlänge des periodischen maximalen Werts und des periodischen minimalen Werts, die im Schritt 1e) bestimmt werden;
1g) Bestimmung des Sauerstoffgehalts ($Sp(O_2)$) des Gewebes ausgehend von den im Schritt 1f) berechneten Verhältnissen bei jeder Wellenlänge;

wobei das Verfahren **dadurch gekennzeichnet ist, dass** es einen Schritt der Validierung oder Invalidierung des im Schritt 1g) bestimmten Sauerstoffgehalts ($Sp(O_2)$) aufweist, und dass im Fall der Invalidierung der Sauerstoffgehalt gemäß einer zweiten Modalität bestimmt wird, wobei die zweite Modalität gemäß einem Verfahren durchgeführt wird, das Gegenstand eines der Ansprüche 1 bis 3 ist.

5. Verfahren nach Anspruch 4, wobei die erste Wellenlänge ($\lambda_1$) zwischen 730 und 750 nm liegt, und wobei die zweite Wellenlänge ($\lambda_2$) zwischen 860 und 880 nm liegt.

6. Verfahren nach einem der Ansprüche 4 oder 5, wobei im Schritt 1b) der Rückstreuabstand ($x_1$) zwischen 6 mm und 8 mm liegt.

7. Verfahren nach einem der Ansprüche 4 bis 6, wobei die interessierende Wellenlänge ($\lambda_i$), die in der zweiten Modalität verwendet wird, entweder die erste Wellenlänge ($\lambda_1$) oder die zweite Wellenlänge ($\lambda_2$) ist.

8. Verfahren nach einem der Ansprüche 4 bis 7, wobei:

   - der Schritt 1a) mit Hilfe einer Lichtquelle (11) durchgeführt wird;
   - der Schritt 1b) mit Hilfe eines elementaren Photodetektors (20j) durchgeführt wird;

   wobei die Lichtquelle und/oder der elementare Photodetektor fest mit einem Bewegungssensor (32) verbunden sind, so dass, wenn eine signifikante Bewegung vom Bewegungssensor erfasst wird, die erste Modalität invalidiert wird.

9. Verfahren nach einem der Ansprüche 4 bis 8, das ebenfalls einen Schritt des Messens einer Herzfrequenz (f) bezüglich des lebenden Körpergewebes aufweist, so dass, wenn die Herzfrequenz oder eine Änderung der Herzfrequenz eine bestimmte Schwelle überschreitet, die erste Modalität invalidiert wird.

10. Oxymetrievorrichtung (1), die gegen den Körper einer Person oder eines Tiers angelegt werden kann, die aufweist:

    - eine erste Lichtquelle ($10_1$), die ein Licht mit einer Wellenlänge zwischen 600 nm und 800 nm emittieren kann;
    - eine zweite Lichtquelle ($10_2$), die ein Licht mit einer Wellenlänge zwischen 800 nm und 1000 nm emittieren kann;
    - mindestens drei elementare Photodetektoren ($20_1$, $20_2$, $20_3$), die sich in drei Abständen ($x_1$, $x_2$, $x_3$), Rückstreuabstände genannt, von einer der Lichtquellen erstrecken, wobei die Rückstreuabstände kleiner als 1,5 cm sind;
    - einen Prozessor, der die Schritte c) bis e) eines Verfahrens nach einem der Ansprüche 1 bis 3 ausgehend von Rückstreustrahlungen durchführen kann, die von jedem elementaren Photodetektor während der Aktivierung der ersten Lichtquelle oder der zweiten Lichtquelle erfasst werden;
    - wobei der Prozessor ebenfalls die Schritte 1c) bis 1g) eines Verfahrens nach einem der Ansprüche 4 bis 9 ausgehend von einer von einem elementaren Photodetektor ($20_1$, $20_2$, $20_3$) während der Aktivierung der ersten Lichtquelle und der zweiten Lichtquelle erfassten rückgestreuten Strahlung durchführen kann.

11. Vorrichtung nach Anspruch 10, die einen Bewegungssensor (32) aufweist, der konfiguriert ist, eine Bewegung der Vorrichtung zu erfassen.

12. Vorrichtung nach Anspruch 10 oder Anspruch 11, wobei die Rückstreuabstände ($x_1$, $x_2$, $x_3$) je zwischen 3 mm und 10 mm liegen.

**Claims**

1. Method for estimating an oxygen content in a living bodily tissue (2), comprising the following steps:

   a) illuminating a surface (2s) of the tissue with an incident light beam (12i), at a wavelength of interest ($\lambda_i$), so as to form an elementary illumination zone (13, 13i), corresponding to the portion of the surface illuminated by the incident light beam;
   b) during the illumination, simultaneously detecting a backscattered emission ($14_1$, $14_2$, $14_3$) emanating from at least three elementary detection zones ($15_1$, $15_2$, $15_3$) on the surface of the sample, each elementary detection zone respectively lying at a distance ($x_1$, $x_2$, $x_3$), called the backscattering distance, from the elementary illumination zone (13, 13i), each backscattering distance being different from the others and smaller than 15 mm;

c) comparing, two by two, the intensities ($M(\lambda_i, x_1)$; $M(\lambda_i, x_2)$; $M(\lambda_i, x_3)$) of the detected backscattered emissions, optionally normalized by an intensity of the incident light beam (12i), and the backscattering distances ($x_1, x_2, x_3$), in order to determine two coefficients ($\alpha(\lambda i)$, $\beta(\lambda i)$);

d) taking into account a first calibration term (*Hbtot*) representing an amount of haemoglobin in the observed tissue, and a second calibration term (Q) characterizing an absorption of the tissue by components of the tissue other than haemoglobin, the first calibration term and the second calibration term being determined in a calibration phase;

e) estimating an oxygen content ($S(O_2)$) of the tissue, on the basis of the comparisons carried out in step c) and of the calibration terms taken into account in step d), step e) comprising applying an expression (12) relating the oxygen content ($S(O_2)$) to the calibration terms taken into account in step d) and to the coefficients obtained in step c), the expression being based on known values of the absorption coefficients of oxyhaemoglobin ($\mu_{aHbO2}(\lambda_i)$) and of the oxyhaemoglobin ($\mu_{aHb}(\lambda_i)$) at the wavelength of interest,

the method being such that the calibration terms taken into account in step d) are determined:

- either in a prior calibration phase, in which an estimation of the oxygen content ($Sp(O_2)$) of the tissue is available, the calibration phase comprising the following steps:

cal-i) implementing steps a) to c), at two different wavelengths ($\lambda_1, \lambda_2$);
cal-ii) using the expression applied in step e) to each wavelength, while considering the estimated oxygen content;

- or in a prior calibration phase comprising the following steps:

cal-i) implementing steps a) to c), at three different wavelengths ($\lambda_1, \lambda_2, \lambda_3$), one of said wavelengths being a so-called isosbestic wavelength, at which the absorption coefficient of oxyhaemoglobin is considered to be equal to the absorption coefficient of deoxyhaemoglobin;
cal-ii) using the expression applied in step e), to each wavelength, obtaining the calibration terms.

- or in a prior calibration phase comprising the following steps:

cal-i) implementing steps a) to c), at three different wavelengths ($\lambda_1, \lambda_2, \lambda_3$);
cal-ii) using the expression applied in step e), to each wavelength, obtaining the calibration terms;

steps c) to e) being implemented using a processor (30).

2. Method according to Claim 1, wherein, in step b), each backscattering distance ($x_1, x_2, x_3$) is larger than 0.3 cm.

3. Method according to any one of the preceding claims, wherein step c) comprises the following substeps:

ci) subtracting the intensities of the backscattered emissions detected at two different backscattering distances ($M(\lambda_i, x_1) - M(\lambda_i, x_2)$; $M(\lambda_i, x_2) - M(\lambda_i, x_3)$);
cii) calculating a logarithm of said subtraction ($ln(M(\lambda_i, x_1) - M(\lambda_i, x_2))$; $ln(M(\lambda_i, x_2) - M(\lambda_i, x_3))$);
ciii) subtracting two different backscattering distances (($x_1 - x_3$), ($x_1 - x_2$)).

4. Bimodal method for determining an oxygen content in a living bodily tissue (2), the method comprising a first determination of an oxygen content in a first mode, via the following steps

1a) illuminating a surface (2s) of the tissue with an incident light beam (12i), at a first wavelength ($\lambda_1$) and a second wavelength ($\lambda_2$), so as to form, during each illumination, an elementary illumination zone (13i), corresponding to the portion of the surface illuminated by the incident light beam;
1b) during each illumination, detecting a backscattered emission emanating from the surface of the sample at a given distance ($x_j$), called the backscattering distance, the backscattering distance being smaller than 15 mm, so as to obtain a backscattered emission respectively detected at the first wavelength ($\lambda_1$) and at the second wavelength ($\lambda_2$);
1c) on the basis of each backscattered emission thus detected, calculating a reflectance ($R(\lambda_1)$, $R(\lambda_2)$) at the first and second wavelength;

1d) reiterating the three steps 1a) to 1c) so as to obtain a temporal variation ($R(\lambda_1,t)$, $R(\lambda_2,t)$) in the reflectance of the tissue at the first and second wavelength;

1e) determining a periodic minimum value ($min(\lambda_1)$, $min(\lambda_2)$) and a periodic maximum value ($max(\lambda_1)$, $max(\lambda_2)$) of the temporal variation ($R(\lambda_1,t)$, $R(\lambda_2,t)$) in the reflectance obtained in step 1d) at each wavelength;

1f) calculating a ratio $\left(\frac{max(\lambda_1)}{min(\lambda_1)}, \frac{max(\lambda_2)}{min(\lambda_2)}\right)$, at each wavelength, of the periodic maximum value and of the periodic minimum value determined in step 1e);

1g) determining the oxygen content ($Sp(O_2)$) of the tissue from the ratios calculated in step 1f) at each wavelength;

the method being **characterized in that** it comprises a step of validating or invalidating the oxygen content ($Sp(O_2)$) determined in step 1g), and **in that**, in case of invalidation, the oxygen content is determined in a second mode, the second mode being implemented according to a method subject of any one of Claims 1 to 3.

5. Method according to Claim 4, wherein the first wavelength ($\lambda_1$) is comprised between 730 and 750 nm, and wherein the second wavelength ($\lambda_2$) is comprised between 860 and 880 nm.

6. Method according to either one of Claims 4 and 5, wherein, in step 1b), the backscattering distance ($x_j$) is comprised between 6 mm and 8 mm.

7. Method according to any one of Claims 4 to 6, wherein the wavelength of interest ($\lambda_i$) used in the second mode is either the first wavelength ($\lambda_1$), or the second wavelength ($\lambda_2$).

8. Method according to any one of Claims 4 to 7, wherein:

- step 1a) is implemented using a light source (11);
- step 1b) is implemented using an elementary photodetector (20j);

the light source and/or the elementary photodetector being securely fastened to a movement sensor (32), such that when a significant movement is detected by the movement sensor, the first mode is invalidated.

9. Method according to any one of Claims 4 to 8, also comprising a step of measuring a cardiac frequency ($f$) relative to the living bodily tissue, such that when the cardiac frequency, or when a variation in the cardiac frequency, crosses a certain threshold, the first mode is invalidated.

10. Oximetry device (1) able to be applied against the body of an individual or of an animal, comprising:

- a first light source ($10_1$) able to emit light at a wavelength comprised between 600 nm and 800 nm;
- a second light source ($10_2$) able to emit light at a wavelength comprised between 800 nm and 1000 nm;
- at least three elementary photodetectors ($20_1$, $20_2$, $20_3$) lying at three distances ($x_1$, $x_2$, $x_3$), called backscattering distances, from one of said light sources, the backscattering distances being smaller than 1.5 cm;
- a processor, able to implement steps c) to e) of a method according to any one of Claims 1 to 3, on the basis of backscattered emissions detected by each elementary photodetector during the activation of the first light source or of the second light source;
- the processor also being able to implement steps 1c) to 1g) of a method according to any one of Claims 4 to 9, on the basis of a backscattered emission detected by an elementary photodetector ($20_1$, $20_2$, $20_3$) during the activation of the first light source and of the second light source.

11. Device according to Claim 10, comprising a movement sensor (32) configured to detect a movement of the device.

12. Device according to Claim 10 or Claim 11, wherein the backscattering distances ($x_1$, $x_2$, $x_3$) are respectively comprised between 3 mm and 10 mm.

**Fig. 1A**

**Fig. 1B**

**Fig. 1C**

**Fig. 2B**

**Fig. 3**

**Fig. 2A**

Fig. 4A

Fig. 4B

Fig. 4C

Fig. 4D

Fig. 4E

Fig. 4F

Fig. 4G

**Fig. 5**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8055321 B

- US 9498134 B

**Littérature non-brevet citée dans la description**

- **HAAHR R.** An Electronic Patch for Wearable Health Monitoring by Reflectance Pulse Oxymetry. *IEEE Transactions on biomedical circuits and systems,* 2011

- **MEGLINSKI, I. et al.** Computer simulation of the skin reflectance spectra computer methods and programs in biomedecine. Elsevier, 2003, vol. 70, 179-186